# EUROPEAN PATENT APPLICATION

(11) **EP 4 497 413 A1**
(43) Date of publication of application: **29.01.2025**
(21) Application number: 24191574.3
(22) Date of filing: 29.07.2024
(51) Int. Cl.: A61F 2/07, A61F 2/06

(54) **AN ENDOLUMINAL PROSTHESIS HAVING A CURVED SIDE TUBE**

(30) Priority: 27.07.2023 AU 2023902394
(71) Applicant: Cook Medical Technologies LLC, Bloomington, IN 47404 (US)
(72) Inventor: KING, Chantelle, Woodridge 4114 (AU); DOHERTY, Rachel, Ingatestone CM4 9HF (GB); CHANDLER, Leticia, Andover SP10 3PA (GB); GUY, James, Seven Hills 4170 (AU); BINSKIN, Robert, Ormiston 4160 (AU)
(74) Representative: Williams Powell

(57) **Abstract**

An endoluminal prosthesis includes a main tubular body having an external surface and defining a main lumen and at least one curved side tube having an internal portion disposed within the main lumen and an external portion extending along a curvilinear path on the external surface of the main tubular body. A portion of an expandable stent attached to the external surface of the main tubular body is disposed radially outward of and attached to the external portion of the curved side tube.

## Description

### Field

The disclosure relates generally to the field of medical devices. More particularly, the disclosure relates to endoluminal prostheses, such as stent-grafts, useful in endovascular treatment of an animal, such as a human being. Example endoluminal prostheses described herein are stent-grafts useful in endoluminal treatment of aneurysms of the aorta. The disclosure also relates to methods of deploying an endoluminal prosthesis in a body vessel of an animal, methods of deploying a main endoluminal prosthesis and a secondary endoluminal prosthesis in a body vessel of an animal, and methods of making an endoluminal prosthesis.

### Background

One form of surgical intervention for weakened, aneurysmal or ruptured vessels involves the use of endoluminal prostheses, such as stent-grafts, to provide some, or all of, the functionality of the original, healthy vessel and/or preserve any remaining vascular integrity. Stent-grafts used in this manner may span the site of the affected vessel. These stent-grafts, or other endoluminal prostheses, may seal off the failed portion of the vessel. These prostheses, for example, are commonly used to treat aneurysms of the aortic arteries.

An endoluminal prosthesis can be of a unitary construction, or can be comprised of multiple prosthetic modules. A modular prosthesis allows a surgeon to accommodate a wide variation in vessel morphology while reducing the necessary inventory of differently sized prostheses. A modular system may accommodate deployment options by allowing the proper placement of one module before the implantation of an adjoining module.

Modular systems are typically assembled in situ by overlapping the tubular ends of the prosthetic modules so that the end of one module sits partially inside the other module, preferably forming circumferential apposition through the overlap region. This attachment process is called "tromboning". The connections between prosthetic modules can be maintained by the friction forces at the overlap region and enhanced by the radial force exerted by the internal prosthetic module on the external prosthetic modules where the two overlap.

When an aneurysm affects a main vessel, it is important to maintain flow to the vessels that branch from the main vessel. The coronary, celiac, superior mesenteric, left common carotid and renal arteries, for example, are branch vessels of the aorta. If these branch vessels are blocked by an endoluminal prosthesis deployed in the aorta, circulation of blood into the branch vessels can be impeded, which can be undesirable. In the endoluminal treatment of aortic aneurysms, for example, maintaining patency of the coronary arteries, which are branch vessels of the ascending aorta, is particularly important. It is common practice, therefore, to cannulate a branch vessel during implantation of an endoluminal prosthesis across a vessel site that includes a branch vessel. A secondary endoluminal prosthesis, such as a secondary graft, can then be deployed into the branch vessel and connected to the main endoluminal prosthesis, such as through a predefined fenestration or side tube on the main endoluminal prosthesis.

While the general anatomical relationship between many main and branch vessels is generally known, some anatomies present one or more branch vessels with an atypical structural relationship to the main vessel. For example, branch vessels may branch from a main vessel at an angle that differs from the angle expected based on general anatomy. Cannulation of these branch vessels during endoluminal treatment of the main vessel with a conventional endoluminal prosthesis can be difficult as typical fenestrations and side branch stubs on these devices are positioned for the expected anatomical relationship between the side branch and main vessel. Also, branch vessels may branch from a main vessel at a location near another anatomical feature that can make cannulation of the branch vessel from the main vessel challenging. Endoluminal treatment of the ascending aorta can involve multiple such challenges, for example. There, one or more coronary arteries may branch from the aorta at an obtuse angle, i.e., in a retrograde direction, and may branch from the aorta at a location that is so close to a heart valve, or a replacement heart valve, that endovascular treatment using known prostheses may not be an option for a particular patient.

### Brief Summary of the Disclosure and Selected Examples

The present invention seeks to provide improved endoluminal prostheses, methods of deploying an endoluminal prosthesis in a body vessel of an animal, methods of deploying a main endoluminal prosthesis and a secondary endoluminal prosthesis in a body vessel of an animal, and/or methods of making an endoluminal prosthesis.

In one aspect, an endoluminal prosthesis comprises a main tubular body defining a main lumen; at least one curved side tube extending from the main tubular body, the side tube defining a side tube lumen having an inlet and an outlet, the inlet in fluid communication with the main lumen, the side tube comprising: an internal portion that extends into the main lumen; and an external portion external to the main tubular body, wherein the external portion of the at least one side tube curves partially around the main tubular body.

In one form, the side tube includes an inlet reinforcement member comprising a first wire ring.

In one form, the side tube includes an outlet reinforcement member comprising a second wire ring.

In one form, the inlet is angled to provide an entrance axis angled at an obtuse angle with respect to a longitudinal axis of the main lumen in a direction that provides a lead-in for a guide wire inserted in a proximal direction through a distal end of the endoluminal prosthesis.

In one form, the internal portion is arranged and constructed to provide a lead-in for a guide wire inserted in a proximal direction through a distal end of the endoluminal prosthesis.

In one form, the endoluminal prosthesis further comprises a first radiopaque marker on the internal portion.

In one form, the endoluminal prosthesis further comprises a second radiopaque marker on the external portion adjacent to the outlet.

In one form, the inlet is a retrograde inlet.

In one form, the side tube includes a first intermediate reinforcement member comprising a third wire ring.

In one form, the side tube includes a second intermediate reinforcement member comprising a fourth wire ring.

In one form, the side tube comprises two pieces of curved graft material sewn together along an inner radius and along an outer radius such that the side tube has a pre-defined curved shape before being secured to the main tubular body of an endoluminal prosthesis. When an endoluminal prosthesis that includes the side tube is deployed within a body vessel, the side tube holds its pre-defined curved shape.

In examples, the inner radius is between 9 mm and 19 mm. In other examples, the inner radius is between 10 mm and 18 mm. In other examples, the inner radius is between 12 mm and 16 mm. In one particular example, the inner radius is about 14 mm.

In examples, the outer radius is between 20 mm and 30 mm. In other examples, the inner radius is between 21 mm and 29 mm. In other examples, the inner radius is between 23 mm and 27 mm. In one particular example, the inner radius is about 25 mm.

In another aspect, an endoluminal prosthesis useful in endoluminal treatment of the aortic arch comprises a main tubular body comprising a biocompatible graft material tube and a plurality of longitudinally spaced-apart self-expanding stents fastened thereto, the main tubular body defining: a main lumen; a pair of curved side tubes extending from main tubular body, each side tube defining a side tube lumen having a retrograde inlet and an outlet, the inlet in fluid communication with the main lumen, each side tube comprising an internal portion that extends into the main lumen; and an external portion external to the main tubular body, wherein the external portion of each side tube curves partially around the main tubular body.

Various example endoluminal prostheses are described.

An example endoluminal prosthesis comprises a main tubular body defining a main lumen and at least one curved side tube extending from the main tubular body, the at least one curved side tube defining a side tube lumen having an inlet and an outlet, the inlet in fluid communication with the main lumen, the side tube comprising an internal portion that extends into the main lumen and an external portion external to the main tubular body extending along a curvilinear path at least partially around the main tubular body.

Another example endoluminal prosthesis comprises a main tubular body having an external surface and defining a main lumen; and at least one curved side tube comprising an internal portion and an external portion and defining a side tube lumen, the internal portion disposed within the main lumen and defining an inlet of the side tube lumen in fluid communication with the main lumen, the external portion extending along a curvilinear path on the external surface of the main tubular body and defining an outlet of the side tube lumen.

Another example endoluminal prosthesis comprises a main tubular body having an external surface and defining a main lumen; at least one curved side tube comprising an internal portion and an external portion and defining a side tube lumen, the internal portion disposed within the main lumen and defining an inlet of the side tube lumen in fluid communication with the main lumen, the external portion extending along a curvilinear path on the external surface of the main tubular body and defining an outlet of the side tube lumen; a first self-expanding stent attached to the external surface of the main tubular body and including a portion disposed radially outward of the external portion of the at least one curved side tube; and a second self-expanding stent attached to the main tubular body and including a portion disposed radially inward of the external portion of the at least one curved side tube.

Another example endoluminal prosthesis comprises a main tubular body having an external surface and defining a main lumen; a first curved side tube on a first side of the main tubular body, the first curved side tube comprising a first internal portion and a first external portion and defining a first side tube lumen, the first internal portion disposed within the main lumen and defining a first inlet of the first side tube lumen in fluid communication with the main lumen, the first external portion extending along a first curvilinear path on the external surface of the main tubular body and defining a first outlet of the first side tube lumen; a second curved side tube on a second side of the main tubular body, the second curved side tube comprising a second internal portion and a second external portion and defining a second side tube lumen, the second internal portion disposed within the main lumen and defining a second inlet of the second side tube lumen in fluid communication with the main lumen, the second external portion extending along a second curvilinear path on the external surface of the main tubular body and defining a second outlet of the second side tube lumen; a first self-expanding stent attached to the external surface of the main tubular body and including a first stent portion disposed radially outward of and attached to the first external portion of the first curved side tube and a second stent portion disposed radially outward of and attached to the second external portion of the second curved side tube; and a second self-expanding stent attached to the main tubular body and including a third stent portion disposed radially inward of the first external portion of the first curved side tube and a fourth stent portion disposed radially inward of the second external portion of the second curved side tube.

Various example methods of deploying an endoluminal prosthesis in a body vessel of an animal are described.

An example method of deploying an endoluminal prosthesis in a body vessel of an animal comprises advancing an end of a delivery system on which an endoluminal prosthesis according to an embodiment is disposed to a point of treatment in a body vessel; rotating the delivery system along its longitudinal axis to align one or more curved side tubes of the endoluminal prosthesis with one or more branch vessels of the body vessel; separating the endoluminal prosthesis from the delivery system; and withdrawing the delivery system from the body vessel.

Various example methods of deploying a main endoluminal prosthesis and a secondary endoluminal prosthesis in a body vessel of an animal are described.

An example method of deploying a main endoluminal prosthesis and a secondary endoluminal prosthesis in a body vessel of an animal comprises advancing an end of a main delivery system on which a main endoluminal prosthesis according to an embodiment is disposed to a point of treatment in the body vessel; rotating the main delivery system along its longitudinal axis to align one or more curved side tubes of the main endoluminal prosthesis with one or more branch vessels of the body vessel; separating the main endoluminal prosthesis from the main delivery system; withdrawing the main delivery system from the body vessel; advancing a guide wire into the lumen of the main endoluminal prosthesis, through the lumen of the curved side tube of the main endoluminal prosthesis, and into the branch vessel; advancing an end of a secondary delivery system on which the secondary endoluminal prosthesis is disposed over the guide wire, through the lumen of the curved side tube of the main endoluminal prosthesis, and into the branch vessel; separating the secondary endoluminal prosthesis from the secondary delivery system; and withdrawing the secondary delivery system from the body vessel.

In examples, the steps of advancing a guide wire, advancing an end of a secondary delivery system, separating a secondary endoluminal prosthesis from the secondary delivery system, and withdrawing the secondary delivery system are repeated using an additional secondary delivery system with an additional secondary endoluminal prosthesis with each repetition. In these examples, another, different curved side tube of the main endoluminal prosthesis can be used during performance of each of the advancing a guide wire and advancing an end of a secondary delivery system steps.

Various example methods of making an endoluminal prosthesis are described.

An example method of making an endoluminal prosthesis comprises forming a first arcuate shape of graft fabric to have a first inner radius and a first outer radius; forming a second arcuate shape of graft fabric to have a second inner radius and a second outer radius; attaching the first arcuate shape to the second arcuate shape along the first and second inner radii and the first and second outer radii to form a curved side tube; passing the curved side tube into an opening in an endoluminal prosthesis precursor such that a portion of the curved side tube is disposed within the lumen of the endoluminal prosthesis precursor and a portion of the curved side tube is external to the lumen of the endoluminal prosthesis precursor; and attaching the curved side tube to the external surface of the endoluminal prosthesis precursor to form an endoluminal prosthesis having a curved side tube.

In examples, the steps of forming a first arcuate shape, forming a second arcuate shape, attaching the first arcuate shape to the second arcuate shape to form a curved side tube, passing the curved side tube into an opening in an endoluminal prosthesis precursor, and attaching the curved side tube to the external surface of the endoluminal prosthesis precursor are repeated, forming and attaching an additional curved side tube with each repetition until a desired number of side tubes is attached to the endoluminal prosthesis precursor to form the final endoluminal prosthesis. Additional understanding of these selected examples and other inventive endoluminal prostheses, methods of deploying an endoluminal prosthesis in a body vessel of an animal, methods of treating an aneurysm, and methods of making an endoluminal prosthesis can be obtained by review of the detailed description of selected examples, below, and the figures.

### Brief Description of the Figures

Selected examples are described below, by way of example only, with reference to the accompanying figures.
Figure 1 is a side view, partially broken away, of an example endoluminal prosthesis.
Figure 2A is a magnified proximal end view of an internal portion of the stent-graft illustrated in Figure 1.
Figure 2B is a cross-sectional view, partially broken away, of the stent-graft illustrated in Figure 1.
Figure 2C is a magnified distal end view of an internal portion of the stent-graft illustrated in Figure 1.
Figure 2D is a is a magnified distal end view of an internal portion of the stent-graft illustrated in Figure 1.
Figure 2E is a side view, partially broken away, of the endoluminal prosthesis illustrated in Figure 1.
Figure 2F is a magnified side view of the endoluminal prosthesis illustrated in Figure 1 showing an outlet of the side tube.
Figure 2G is a is a magnified distal end view of an internal portion of the stent-graft illustrated in Figure 1.
Figure 2H is a side view, partially broken away, of an alternative endoluminal prosthesis with an alternative side tube.
Figure 3A is a plan view of a partially constructed curved side tube portion of the endoluminal prosthesis illustrated in Figure 1.
Figure 3B is an isometric view of a partially constructed curved side tube portion of the endoluminal prosthesis illustrated in Figure 1.
Figure 3C is an isometric view of a partially constructed curved side tube portion of the endoluminal prosthesis illustrated in Figure 1.
Figure 3D is an isometric view of a fully constructed curved side tube portion of the endoluminal prosthesis illustrated in Figure 1.
Figure 4A is a side view of the endoluminal prosthesis illustrated in Figure 1. The endoluminal prosthesis is illustrated in a first rotational position relative to a lengthwise axis of the endoluminal prosthesis to illustrate the side tubes of the endoluminal prosthesis.
Figure 4B is a side view of the endoluminal prosthesis illustrated in Figure 1. The endoluminal prosthesis is illustrated in a second rotational position relative to a lengthwise axis of the endoluminal prosthesis to illustrate a side tube of the endoluminal prosthesis. The endoluminal prosthesis is rotated 90 degrees with respect to the view illustrated in Figure 4A.
Figure 4C is a side view of the endoluminal prosthesis illustrated in Figure 1. The endoluminal prosthesis is illustrated in a third rotational position relative to a lengthwise axis of the endoluminal prosthesis to illustrate the side tubes of the endoluminal prosthesis. The endoluminal prosthesis is rotated 180 degrees with respect to the view illustrated in Figure 4A.
Figure 5 is a side view of the endoluminal prosthesis illustrated in Figure 1 loaded onto a delivery device.
Figure 6 is a schematic illustration of a human aortic arch with the endoluminal prosthesis illustrated in Figure 1 in a first deployment stage within the aortic arch.
Figure 7 is a schematic illustration of a human aortic arch with the endoluminal prosthesis illustrated in Figure 1 deployed within the aortic arch and a guide wire positioned for cannulation of a coronary artery branched from the aorta.
Figure 8 is a schematic illustration of a human aortic arch with the endoluminal prosthesis illustrated in Figure 1 deployed within the aortic arch and a branch vessel prosthesis deployed within a coronary artery and secured to a curved side tube of the endoluminal prosthesis.
Figure 9 is a side view of another example endoluminal prosthesis.
Figure 10 is a schematic illustration of a human aortic arch with the endoluminal prosthesis illustrated in Figure 9 deployed within the aortic arch and a branch vessel prosthesis deployed within a coronary artery and secured to a curved side tube of the endoluminal prosthesis.
Figure 11 is a flowchart representation of an example method of deploying an endoluminal prosthesis in a body vessel of an animal.
Figure 12 is a flowchart illustration of an example method of deploying a main endoluminal prosthesis and a secondary endoluminal prosthesis in a body vessel of an animal.
Figure 13 is a flowchart representation of an example method of making an endoluminal prosthesis.

### Detailed Description of Selected Examples

The following detailed description and the appended drawings describe and illustrate various example endoluminal prostheses, methods of deploying an endoluminal prosthesis in a body vessel of an animal, methods of deploying a main endoluminal prosthesis and a secondary endoluminal prosthesis in a body vessel of an animal, and methods of making an endoluminal prosthesis. The description and illustration of these examples are provided to enable one skilled in the art to make and use example endoluminal prostheses, such as stent-grafts, useful in endovascular treatment of an animal, such as endoluminal treatment of an aortic aneurysm in a human being, and to perform example methods of deploying an endoluminal prosthesis in a body vessel of an animal, methods of deploying a main endoluminal prosthesis and a secondary endoluminal prosthesis in a body vessel of an animal, and methods of making an endoluminal prosthesis.. The inclusion of detailed descriptions and illustrations of these examples is not intended to limit the scope of the invention, or its protection, in any manner. The invention is capable of being practiced or carried out in various ways and the examples described and illustrated herein are not considered exhaustive.

As used herein, the terms 'comprise' and 'include' and variations such as 'comprising' and 'including' imply the inclusion of a stated integer or group of integers but not the exclusion of any other integer or group of integers.

As used herein, the term "distal," with respect to a portion of a blood vessel, refers to the portion of the blood vessel further from the heart with respect to the direction of blood flow away from the heart. With respect to a prosthesis or deployment device, the term "distal" refers to the portion of the prosthesis or deployment device further from the heart with respect to the direction of blood flow away from the heart through a body vessel within which the prosthesis or deployment device is disposed or intended to be disposed.

As used herein, the term "proximal," with respect to a portion of a blood vessel, refers to the portion of the blood vessel closer to the heart with respect to the direction of blood flow away from the heart. With respect to a prosthesis or deployment device, the term "proximal" refers to the portion of the prosthesis or deployment device closer to the heart with respect to the direction of blood flow away from the heart through a body vessel within which the prosthesis or deployment device is disposed or intended to be disposed.

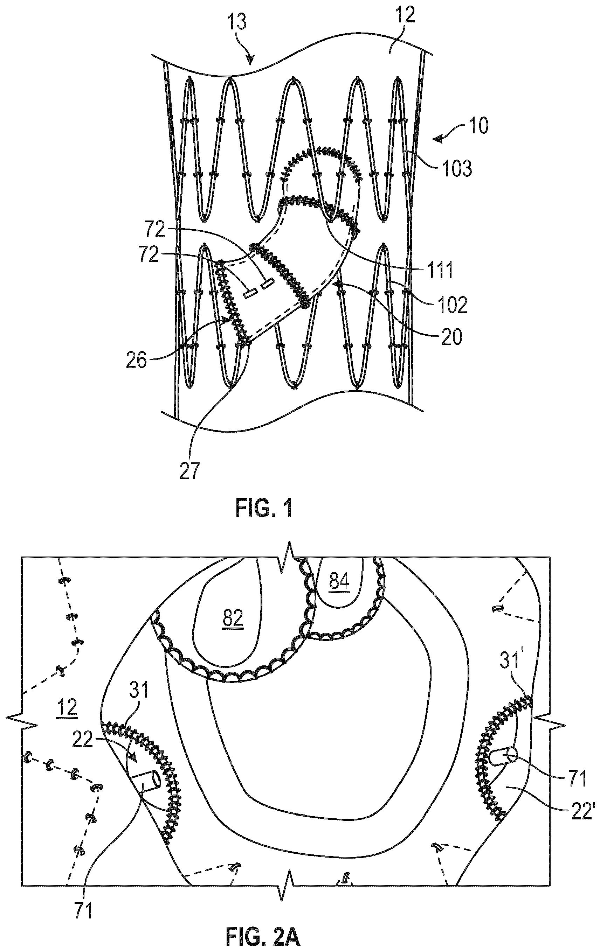

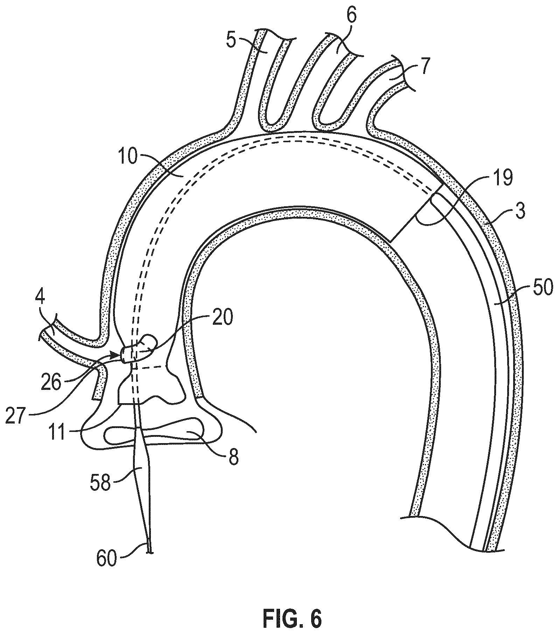

Each of Figures 1, 2A, 2B, 2C, 2D, 2E, 2F, 4A, 4B, 4C, 5, 6, 7, and 8 illustrate a first example endoluminal prosthesis 10. The endoluminal prosthesis 10 is a stent-graft useful in the endoluminal treatment of body vessels. For example, as illustrated in Figures 6, 7, and 8, the endoluminal prosthesis 10 is useful in the endoluminal treatment of the aorta. As described in detail below, and as reflected in Figures 6, 7, and 8, endoluminal prosthesis 10 is particularly well-suited for endoluminal treatment of the ascending aorta, particularly with one or more coronary arteries that branch from the ascending aorta at an obtuse angle (i.e., in a retrograde direction).

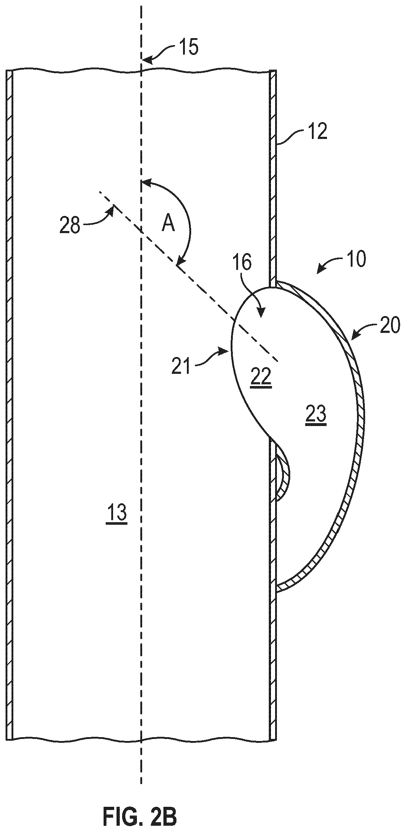

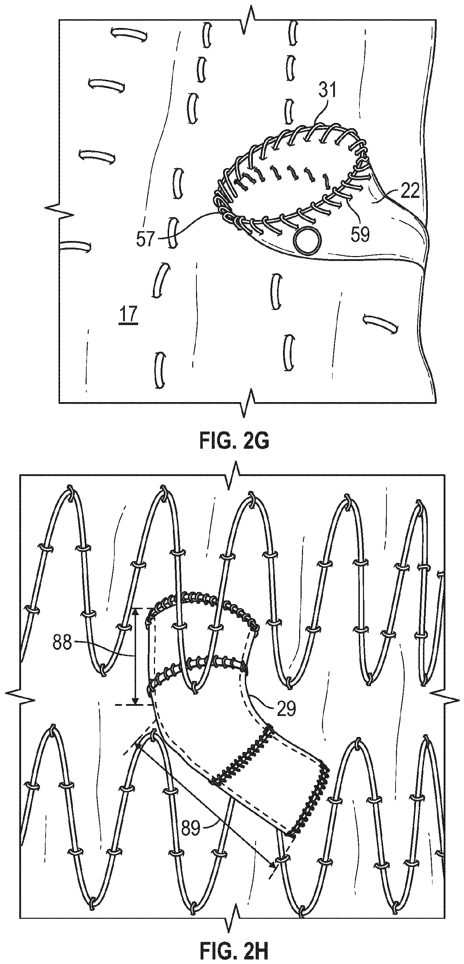

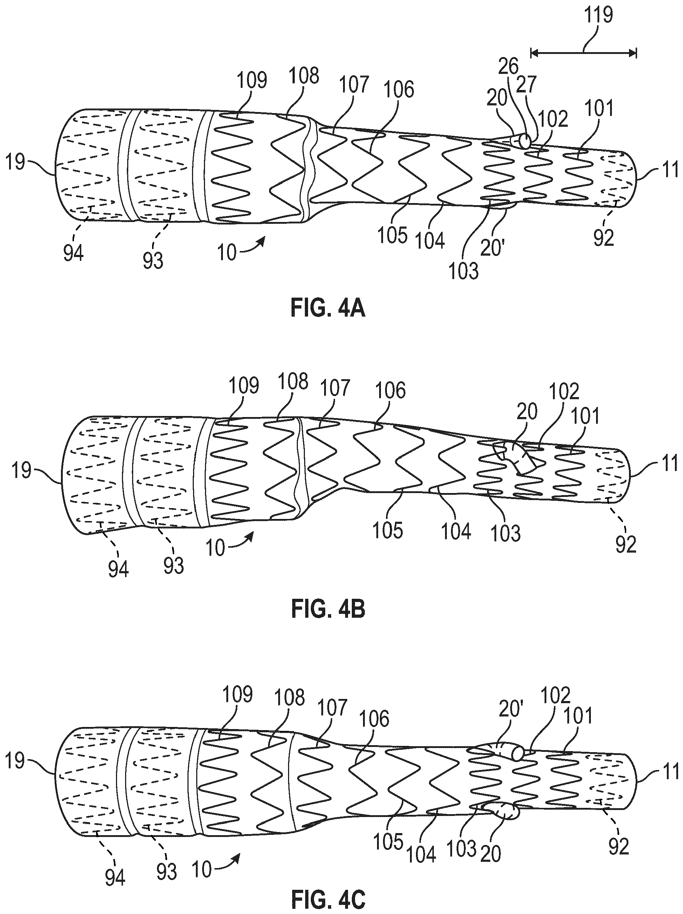

The endoluminal prosthesis 10 comprises a main tubular body 12 defining a main lumen 13. The main lumen 13 is most clearly shown in Figure 2B. Referring to Figures 1 and 2B, it can be seen that the endoluminal prosthesis 10 further comprises at least one curved side tube 20 extending from main tubular body 12. As best illustrated in Figures 4A and 4C, endoluminal prosthesis 10 includes first 20 and second 20' curved side tubes disposed on opposing sides of the endoluminal prosthesis with respect to a longitudinal axis of the endoluminal prosthesis. The inclusion of first 20 and second 20' curved side tubes positioned on the endoluminal prosthesis in this manner make the endoluminal prosthesis 10 particularly well-suited for use in endoluminal treatment of an ascending aorta that includes two coronary arteries that branch from the ascending aorta at an obtuse angle A. Other embodiments of endoluminal prostheses, though, include only a single curved side tube, or more than two curved side tubes. Indeed, endoluminal prostheses according to particular embodiments include three or more curved side tubes. In an endoluminal prosthesis according to an embodiment that includes two or more curved side tubes, the curved side tubes may have identical structures or may be different. For example, the curved side tubes can have the same or different lengths, the same or different inner radii, the same or different outer radii, and the same or different circumference at the inlets and outlets or the curved side tubes can follow the same or different (for example, clockwise or counter-clockwise) curvilinear paths on the external surface of the main tubular body. For simplicity, a single side tube will be described below.

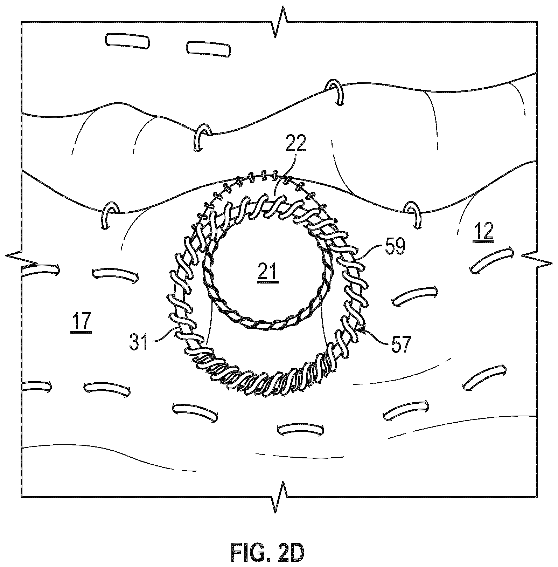

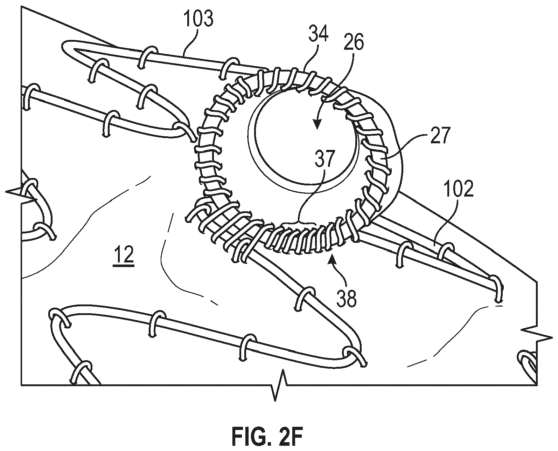

The side tube 20 defines a side tube lumen 23 having an inlet 21 and an outlet 26. The inlet 21 is in fluid communication with the main lumen 13. The side tube lumen 23 comprises an internal portion 22 and an external portion 25. The internal portion 22 extends into the main lumen 13, for example, as can be seen in Figs.. 2A, 2B, 2D, and 2F. Inclusion of internal portion 22, as opposed to an ostium opening into a side tube, is considered advantageous at least because the internal portion 22 facilitates cannulation of a branch vessel through the side tube 20. The internal portion 22 provides an internal lead into the side tube 20 and helps to stabilize the side tube 20 during cannulation of a branch vessel, such as a coronary artery, through the side tube 20, for example. As best illustrated in Fig. 2F, the internal potion 22 in the example endoluminal prosthesis 10 includes an inlet reinforcement member that includes wire ring 31. A circumferential portion 57 of the wire ring 31 is attached to the internal surface of the main tubular body 12. The remaining circumferential portion 59 of the wire ring 31 is free of the internal surface 17 of the main tubular body 12. Also, as best illustrated in Figs. 2D and 2F, the wire ring 31 is disposed at a non-orthogonal and non-parallel angle to the longitudinal axis 15 of the main tubular body 12. Advantageously, as best illustrated in these Figures, the wire ring 31 extends proximally from the circumferential portion 57 of the wire ring 31 that is attached to the internal surface 17 of the main tubular body 12 to the circumferential portion 59 of the wire ring 31 is free of the internal surface 17 of the main tubular body 12. This structural arrangement is considered advantageous at least because it facilitates entry into the side tube 20 by both proximally directed medical devices, such as a wire guide, and distally directed fluid flow.

The external portion 25 of the side tube 20 is external to the main tubular body 12. The external portion 25 extends along the external surface of the main tubular body and curves partially around the main tubular body 12, as can be seen in Figure 1. This results in the outlet 26 facing in an approximately circumferential or tangential direction with respect to the main tubular body 12. This provides advantages once deployed within a vessel such as the aorta. For instance, it provides a degree of flexibility in bridging across to a branch vessel, with a guide wire for example, when alignment with the branch vessel is imprecise or difficult to judge before a procedure. In the illustrated example endoluminal prosthesis 10, side tube 20 extends along the main tubular body 12. In other embodiments, one or more side tubes can be included that extend distally along main tubular body 12. Also in other embodiments, one or more side tubes can be includes that extend substantially perpendicularly to a lengthwise axis of the main tubular body can be included.

In the illustrated example endoluminal prosthesis 10, side tube 20 has a curvilinear shape. While a curvilinear shape is considered advantageous, any suitable shape can be used for a side tube in an endoluminal prosthesis according to a particular embodiment. A skilled artisan will be able to select a suitable shape for a particular side tube based on various considerations, including the angle at which a particular side branch branches from a main body vessel within which the endoluminal prosthesis is intended to be implanted. Fig. 2H illustrates an alternative endoluminal prosthesis 10' that includes a side tube 29'that includes a first portion 88' that extends from the main tubular body 12' in a direction substantially parallel to the longitudinal axis of the main tubular body and a second portion 89' that extends from the first portion 88' along a curvilinear path partially around the main tubular body 12'.

The inlet of the side tube 20 includes an inlet reinforcement member. The outlet of the side tube 20 includes an outlet reinforcement member. The inlet reinforcement member may comprise a first wire ring 31 and the outlet reinforcement member comprising a second wire ring 32. These wire rings may comprise a planar circular ring of wire for example. The wire rings may comprise a plurality of turns of the wire. For example, the wire rings may comprise three wraps of the wire with the wire ends terminating in small loops known as 'ears'. The wire rings may comprise a shape memory wire such as a nitinol wire, a radiopaque wire formed of materials having radiopaque characteristics such as gold, silver, or platinum, or may be a composite of a shape memory wire and a radiopaque wire. One or both of the inlet and outlet reinforcement members may be formed of other materials configured to provide resilience. These terminal ears eliminate or at least reduce the chance of damage to a vessel wall in use. The side tube 20 further includes a first and a second intermediate reinforcement member comprising a third wire ring 32 and a fourth wire ring 33 respectively. In other embodiments there may be less, or more, intermediate reinforcement members provided.

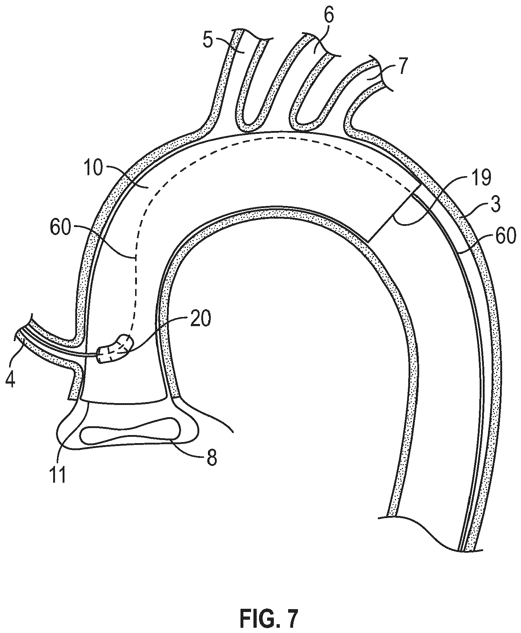

Again, referring to Figure 2B, it can be seen that the inlet 21 of the side tube 20 is angled to provide an entrance axis 28 angled at an obtuse angle A with respect to a longitudinal axis 15 of the main lumen in a direction that provides a lead-in for a guide wire 60 inserted in a proximal direction through a distal end 19 of the endoluminal prosthesis. Figure 7 shows a guide wire 60 that has been led into the inlet 21 of the side tube lumen 23 facilitating cannulation of the coronary artery 4. The inlet 21 is a retrograde inlet. That is, its internal portion extends in a direction that requires blood to flow in a reverse direction to enter the side tube 20.

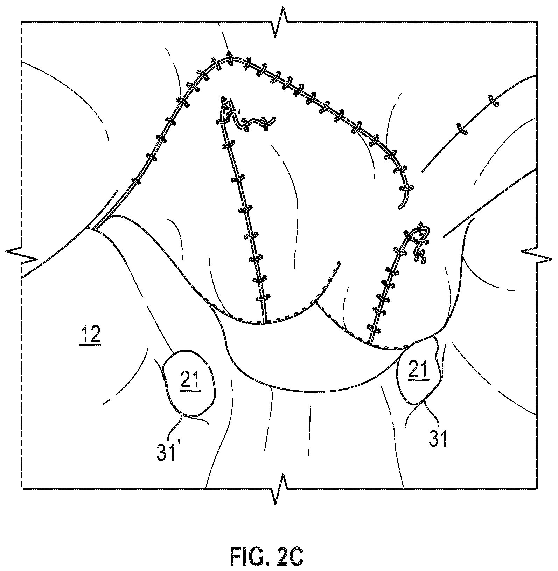

Referring to Figure 2A, a magnified view of an internal portion 22 of the endoluminal prosthesis 10 of Figure 1 looking distally from the proximal end 11 of the endoluminal prosthesis 10, it can be seen that the internal portion 22 is arranged and constructed to provide a lead-in for a guide wire 60, such as the guide wire 60 shown in Figure 7, inserted in a proximal direction through a distal end 19 of the endoluminal prosthesis 10. The inlet 21 of the side tube lumen 23 can be seen in Figure 2C, a magnified view of an internal portion of the endoluminal prosthesis 10 of Figure 1 looking from a distal end. This is shown even more clearly in the magnified view of an internal portion of the endoluminal prosthesis 10 of Figure 1 looking from a distal end, as illustrated in Figure 2D.

Specifically, the retrograde orientation of inlet 21 facilitates passage of a guide wire 60 into the side tube lumen 23 and, as best illustrated in Figure 7, into a coronary artery 4 branched from the ascending aorta an obtuse angle B. The structural orientation of the inlet 21, and indeed of side tube 20, enables a user to use a guide wire 60 to cannulate from a downstream arterial access point into the inlet 21 of the side tube lumen 23. For instance, the guide wire 60 may be introduced into a femoral artery of the patient using known endovascular techniques.

Referring to Figures 1 and 2A, a first radiopaque marker 71 on the internal portion 22 of the side tube 20 is provided. A second radiopaque marker 72 is provided on the external portion adjacent to the outlet of the side tube 20. These markers 71, 72 assist a surgeon by allowing the position of the internal portion 22 and the external portion to be seen under fluoroscopy. Standard radiopaque gold markers are preferably used to assist graft orientation when the prosthesis is viewed through a fluoroscope. An endoluminal prosthesis according to other embodiments may utilize radiopaque wires to mark the region, for example, the hole or fenestration, where a curved side tube 20 is attached to the main tubular body 12. Radiopaque wires may also be provided along the spine of the curved side tube 20 to help demonstrate the directionality of the side tube 20. In embodiments with multiple tubes, the tubes may be provided with different markers or have a marker difference to help differentiate between the tubes, for example, under fluoroscopy.

The main tubular body 12 of the endoluminal prosthesis 10 comprises a biocompatible graft material tube and a plurality of longitudinally spaced-apart self-expanding stents fastened thereto. The specific stenting arrangement of this particular embodiment is shown on Figures 4A, 4B, and 4C and includes internal stents 92, 93 and 94 and external stents 101, 102, 103, 104, 105, 106, 107, 108 and 109. More specifically, endoluminal prosthesis 10 has three internal stents 92, 93 and 92 shown in dashed lines and a plurality of external stents 101, 102, 103, 104, 105, 106, 107, 108 and109along the length of its tubular body. External stents 101, 102, 103, 104, 105, 106, 107, 108 and 109 are intermediate stents and internal stents 30 and 90 are end stents. The internal stent 92 is at the proximal end 11 and acts on a sealing zone also at the proximal end 11. The internal stents 93, 94 are at the distal end 19 and act on a sealing zone also at the distal end 19. The stenting arrangement shown in Figures 4A, 4B, and 4C are exemplary only, and it is understood that other suitable stenting arrangements, including, for example, the utilization of external stents at the distal end and/or the proximal end, may be used based on various considerations, including patient anatomy and necessity of fixation.

Advantageously, as best illustrated in Figures 1, 2E, 2F, and 5, at least one external stent 103 extends over side tube 20. That is, at least one external stent 103 has a portion that is disposed radially outward of the side tube 20, 20'. Also, in embodiments with multiple side tubes, such as side tubes 20, 20' in example endoluminal prosthesis 10, at least one external stent 103 extends over each of the side tubes 20, 20'. This structural relationship between external stent 103 and the side tubes 20, 20' is considered advantageous at least because it allows the external stent 103 to function as an external stent for each of the main tubular body 12 and the curved side tubes 20, 20' of the endoluminal prosthesis 10. In these embodiments, a structural arrangement in which the same external stent extends over all curved side tubes in the endoluminal prosthesis is considered particularly advantageous, but a single external stent can extend over any number of the curved side tubes in the endoluminal prosthesis, including only one, more than one, two, more than two, and less than all of the curved side tubes in the endoluminal prosthesis. Furthermore, as best illustrated in Figure 1, a structural arrangement in which an apex 111 of the external stent 103 that extends over the curved side tube is positioned radially outward of and on the curved side tube 20. While inclusion of at least one external stent 103 that extends over side tube 20 is considered advantageous, this structural arrangement is considered optional and, indeed, a side tube can extend between struts of a stent such that no portion of a stent extends over the side tube.

Also as best illustrated in Figures 1, 2E, 2F, and 5, a structural arrangement in which a curved side tube 20 spans two adjacent external stents 102, 103 is considered advantageous, particularly with one external stent 103 extending over the side tube 20 and the other external stent 102 extending beneath the side tube 20. That is, one external stent 103 of the adjacent pair of external stents 102, 103 includes a portion that is radially outward of and over the curved side tube 20 and the other external stent 102 of the adjacent pair of external stents 102, 103 includes a portion that is radially inward of and under the curved side tube 20. This structural arrangement is considered advantageous at least because it leverages the stent structure of the main tubular body to provide support for the curved side tube 20.

As best illustrated in Figures 4A, 4B, and 4C, multiple stents 92, 101, 102 are positioned axially between the curved side tubes 20, 20' and the proximal end 11 of the endoluminal prosthesis 10, including two external stents 101, 102 and one internal stent 92. Any suitable number of stents can be disposed axially between a curved side tube and the proximal end of an endoluminal prosthesis according to a particular embodiment, and a skilled artisan will be able to select a suitable number of stents to be disposed axially between a curved side tube and the proximal end of an endoluminal prosthesis based on various considerations, including a distance between the intended landing zone for the proximal end of the endoluminal prosthesis and the location of a branch vessel intended to be cannulated through the curved side tube, which can be a distance determined through measurement or other analysis of an image or images of the specific body vessel within which the endoluminal prosthesis is intended to be implanted. Inclusion of at least one stent axially between a curved side tube and the proximal end of an endoluminal prosthesis is considered particularly advantageous at least because such a stent can be positioned on the main tubular body of the endoluminal prosthesis to provide support to both the proximal end of the endoluminal prosthesis and one or more curved side tubes of the endoluminal prosthesis. It is also considered advantageous to include at least two stents axially between a curved side tube and the proximal end of an endoluminal prosthesis, with the proximal most stent being an internal stent and the distal most stent being an internal stent, at least because this arrangement provides the benefits of a terminal internal stent, as described above, and those of an external stent positioned radially inward of and beneath the curved side tube, also as described above. Endoluminal prostheses according to these embodiments are considered particularly well-suited for use in endoluminal treatment of vessels in which the intended landing zone of the proximal end of the stent is adjacent an anatomical structure of the vessel not to be spanned by the endoluminal prosthesis, such as a valve. For example, as best illustrated in Figures 6, 7, and 8, the example endoluminal prosthesis 10 is particularly well-suited for deployment in the aortic arch in a manner that positions the proximal end 11 of the endoluminal prosthesis 10 in the aortic root and adjacent the aortic valve 8.

Also, as best illustrated in Figure 4A, the distance 119 between the proximal edge 27 of the outlet 26 of the side tube 20 and the proximal end 11 of the endoluminal prosthesis is advantageously less than 25% of the axial length of the endoluminal prosthesis 10. An endoluminal prosthesis according to a particular embodiment can have any suitable distance between the proximal edge of the outlet of the curved side tube and the proximal end of the endoluminal prosthesis, and a skilled artisan will be able to select a suitable distance based on various considerations, including a distance between the intended landing zone for the proximal end of the endoluminal prosthesis and the location of a branch vessel intended to be cannulated through the curved side tube, which can be a distance determined through measurement or other analysis of an image or images of the specific body vessel within which the endoluminal prosthesis is intended to be implanted. Examples of suitable distances between the proximal edge of the outlet of a curved side tube and the proximal end of the endoluminal prosthesis include, but are not limited to, less than 30% of the axial length of the endoluminal prosthesis, less than 25% of the axial length of the endoluminal prosthesis, less than 20% of the axial length of the endoluminal prosthesis 10, less than 15% of the axial length of the endoluminal prosthesis, less than 10% of the axial length of the endoluminal prosthesis, and less than 5% of the axial length of the endoluminal prosthesis. For endoluminal prostheses intended to be implanted in the aortic root and used for cannulation of coronary arteries, a distance less than 25% but greater than 10% of the axial length of the endoluminal prosthesis is considered particularly advantageous. Also, a distance less than 20% but greater than 15% of the axial length of the endoluminal prosthesis is considered particularly advantageous. For a deployment where the intended landing zone for the distal end of the endoluminal prosthesis is proximal to the brachiocephalic artery 5, the left common carotid artery 6, and/or the left subclavian artery 7, a distance between the proximal edge of the outlet of a curved side tube and the proximal end of the endoluminal prosthesis may be greater than 30% but less than 50% of the axial length of the endoluminal prosthesis. Also, a distance between the proximal edge of the outlet of a curved side tube and the proximal end of the endoluminal prosthesis may be based on data relating to one or both of a main vessel and a branch vessel in a patient into which the endoluminal prosthesis is intended to be implanted. In some examples, a distance between the proximal edge of the outlet of a curved side tube and the proximal end of the endoluminal prosthesis that is greater than 50% but less than 70% of the axial length of the endoluminal prosthesis may be desirable.

The biocompatible graft material tube can be made from numerous materials, including both synthetic polymers and natural tissues. Preferably, the graft tube material 20 is formed from a biocompatible material that is substantially non-toxic in the in vivo environment of its intended use and substantially unrejected by the patient's physiological system (i.e., is nonantigenic). The graft tube material may be made of any of the materials described in U.S. Pat. No. 7,407,509 to Greenberg et al. or U.S. Patent Application Pub. No. 2009/0171451 to Kuppurathanam et al., which are incorporated herein by reference in their entirety.

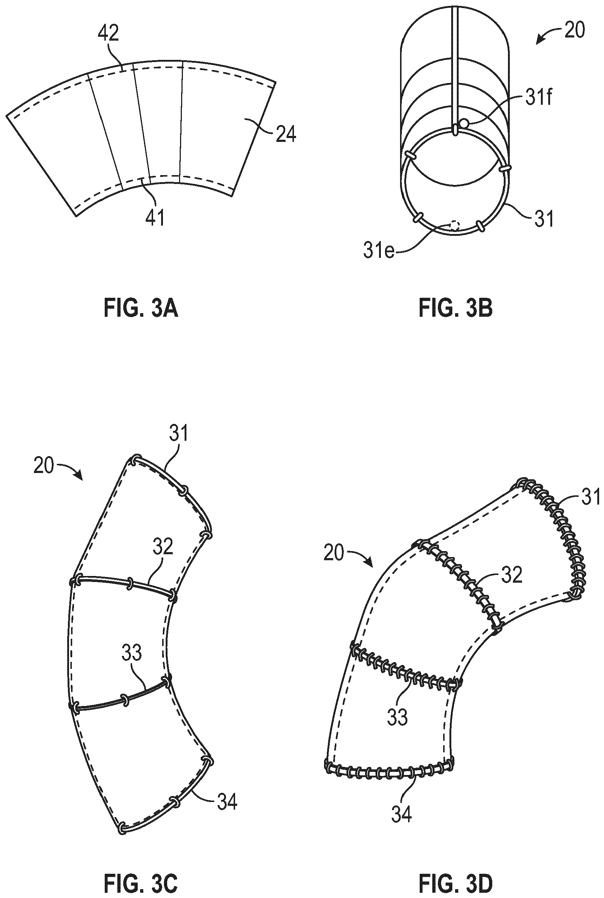

The curved side tube is advantageously fabricated from the same graft tube material used for main tubular body of the endoluminal prosthesis 10. Figures 3A, 3B, 3C, and 3D illustrate the curved side tube in various stages of an example fabrication process.

Two pieces of graft fabric are cut to the arcuate shapes shown in Figure 3A. This figure shows two fabric sheets lying flat, one on top of the other and then sewn together along an inner radius 41 and an outer radius 42. At this stage of the fabrication, a defined curvature is set. The defined curvature can be varied to suit particular anatomies and procedures but will fall within the range as described below. The same construction can be arrived at with a tubular fabric that is laid out flat and then sewn to achieve the inner radius 41 and outer radius 42 shown in Figure 3A, after which excess fabric can be trimmed. With either construction method, the result is two sheets of fabric that are held together by stitching along an inner radius 41 and an outer radius 42, thereby providing a side tube 20 having a defined curvature. It is noted that the specific stitching illustrated in the Figures is exemplary in nature, and any suitable stitching can be used. For example, stitching around stent portions can be double stitching, with two stitches disposed side by side along the relevant stitching path.

Any suitable inner radius and any suitable outer radius can be used for a side tube in an endoluminal prosthesis according to an embodiment, and a skilled artisan will be able to select a suitable inner radius and a suitable outer radius for a side tube in an endoluminal prosthesis according to a particular embodiment based on various considerations, including an angle at which a branch vessel intended to be cannulated by use of the side tube branches from a main vessel within which the endoluminal prosthesis is intended to be used. In some embodiments, one or both of the inner radius and outer radius is based on data relating to one or both of a main vessel and a branch vessel in a patient into which the endoluminal prosthesis is intended to be implanted. Inclusion of one or more of an inner radius and an outer radius that provides a radius of curvature for the side tube that provides a gradual curve that avoids kinking upon deployment in a main vessel is considered advantageous. As noted above, example endoluminal prosthesis 10 is particularly well-suited for endoluminal treatment of the ascending aorta of a human from which one or both coronary arteries branch from the aorta at an oblique angle, such as the aorta 3 illustrated in Figure 7. For endoluminal prostheses intended for implantation in an ascending aorta for such endoluminal treatment, an inner radius between 9 mm and 19 mm is considered advantageous. Also for these embodiments, an inner radius between 10 mm and 18 mm is considered advantageous. Also for these embodiments, an inner radius between 12 mm and 16 mm is considered particularly advantageous. In one particular example, an inner radius of about 14 mm is considered particularly advantageous. Also for these embodiments, an outer radius between 20 mm and 30 mm is considered advantageous. Also in these embodiments, an outer radius between 21 mm and 29 mm is considered advantageous. Also for these embodiments, an outer radius between 23 mm and 27 mm is considered particularly advantageous. In one particular example, an outer radius of about 25 mm is considered particularly advantageous. A side tube with a curvature that is considered particularly advantageous for inclusion in an endoluminal prosthesis intended for endoluminal treatment of the ascending aorta of a human from which at least one coronary artery branches from the aorta at an oblique angle has an inner radius between 9 mm to 19 mm and an outer radius of between 20 mm and 30 mm. An endoluminal prosthesis with first and second side tubes, positioned on opposite sides of the endoluminal prosthesis with respect to a longitudinal axis of the endoluminal prosthesis, each having an inner radius between 9 mm to 19 mm and an outer radius of between 20 mm and 30 mm is particularly well-suited for endoluminal treatment of the ascending aorta of a human from which both coronary arteries branch from the aorta at an oblique angle. In order to provide patency in the finished side tube 20, reinforcement members are provided, for example, by providing wire rings as shown in Figures 3B and 3C. As shown in Figure 3B, an inlet reinforcement member in the form of a first wire ring is placed around the newly sewn flat tube shown in Figure 3A with each end ear being sewn on one of the two pieces of fabric so two ears, 31e, 31f are on one side and neither ear is on the other side. The result of this step is shown in Figure 3B, held by tacking stitches.

Now referring to Figure 3C, the result of further steps of tack stitching, an outlet reinforcement member in the form of a second wire ring 34 is shown. Also, first and second intermediate reinforcement members comprising a third wire ring 32 and a fourth wire ring 33 respectively are shown tacked in place.

Figure 3D shows the result of a further step where each of the rings are blanket stitched onto the graft material. The curved side tube 20 is now ready to be attached to the main tubular body 12 to achieve the constructions shown, as is shown in Figure 1. The finished curved side tube 20 is now configured to hold a defined curvature and, after release from a compressed state within a deployment device, the wire rings 31, 32, 33, 34 will facilitate patency and therefore blood flow.

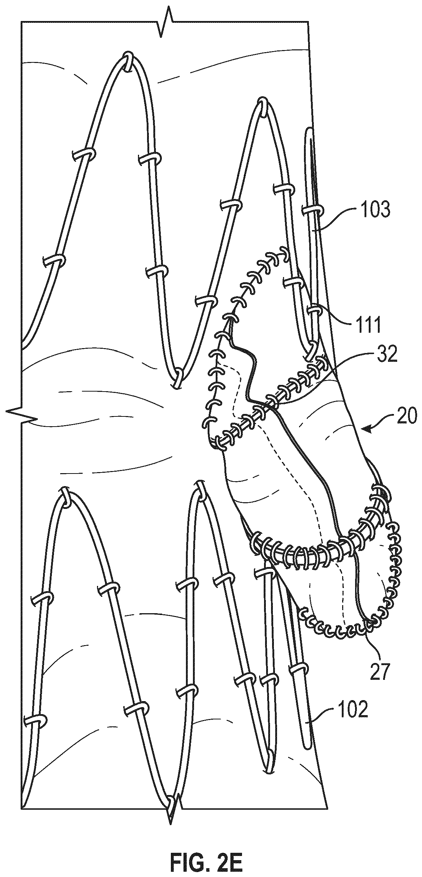

The finished curved side tube 20 is attached to the main tubular body 12 through a hole or fenestration having an oval or egg shape by stitching as is shown in Figure 2E.

Referring to Figures 2E and 2F, which show the side tube 20 and the outlet 26 of the side tube 20, it can be seen that the side tube is supported by the zig-zag stent 103, the second wire ring 32 and an attachment 38 to the main tubular body 12. The overlap of the zig-zag stent 103 and the second wire ring 32 assist in stabilising the position of the side tube 20 and providing patency once deployed.

The attachment 38 to the main tubular body 12 shown in Figure 2F, by blanket stitching for instance, holds the outlet 26 in a known position with respect to the main tubular body which assists in planning and executing endovascular repair.

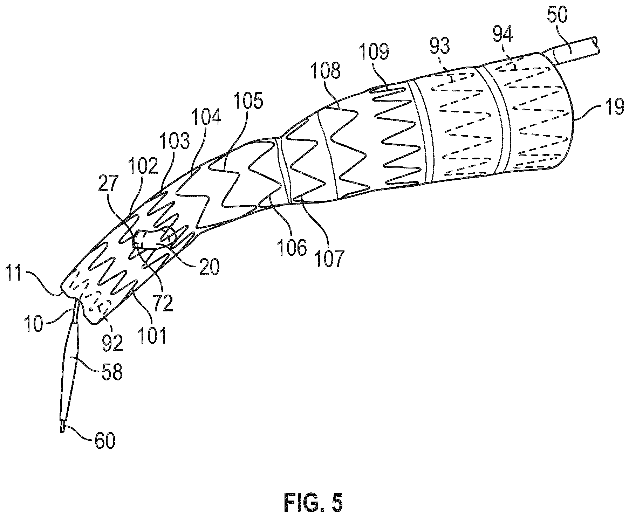

Referring now to Figure 5, the endoluminal prosthesis is shown around a delivery device 45 which includes a pusher 50, a cannula 10, and a tip 58. The delivery device 45 is disposed over a previously placed guide wire 60 and has been advanced along the guide wire 60 in a conventional manner.

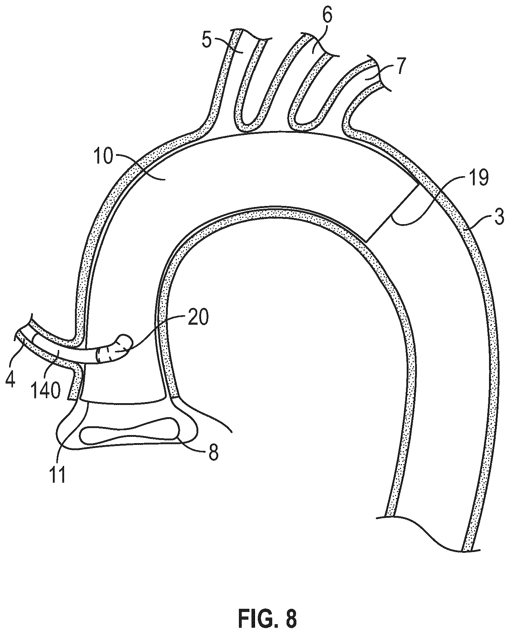

Figures 6, 7 and 8 are progressive illustrations of deployment of a thoracic aortic arch endoluminal prosthesis 10 into the aorta of a patient. Each of these figures shows use of example endoluminal prosthesis 10 to treat an aneurysm in the ascending aorta and near the locations at which the coronary arteries branch from the aorta. Furthermore, Figure 7 illustrates cannulation of a coronary artery 4 through curved side tube 20 and Figure 8 illustrates a secondary endoluminal prosthesis 140, which is an expandable stent, that has been deployed in coronary artery 4 by being passed over guide wire 60 and released from a secondary delivery system (not illustrated). secondary endoluminal prosthesis 140 is positioned within the vessel such that its proximal end overlaps the outlet 26 of side tube 20 of endoluminal prosthesis 10. In this manner, endoluminal prosthesis 10 is useful in the endoluminal treatment of an aortic aneurysm and the attendant stenting of one or more coronary arteries at the point at which the one or more coronary arteries branch from the ascending aorta. Also in this manner, and as illustrated in Figures 6, 7, and 8, endoluminal prosthesis 10 is particularly well suited for endoluminal treatment of an aortic aneurysm and the attendant stenting of one or more coronary arteries that branch from the ascending aorta at an obtuse angle, i.e., in a retrograde direction.

Referring to Figure 6, the endoluminal prosthesis 10 is shown partially deployed in the aortic arch with its proximal end 11 adjacent to the location of the aortic valve 8 and its distal end 19 in the aortic arch. Figure 6 also shows the outlet 26 of the side tube 20 roughly aligned with the coronary artery 4.

Figure 8 shows the endoluminal prosthesis 10 fully expanded in position in the aortic arch and shows cannulation of the coronary artery 4 using a guide wire 60. Finally, Figure 8 shows a branch vessel prosthesis 140 deployed through the side tube 20. The embodiment described and illustrated may be particularly suitable for use alongside a transcatheter aortic valve replacement.

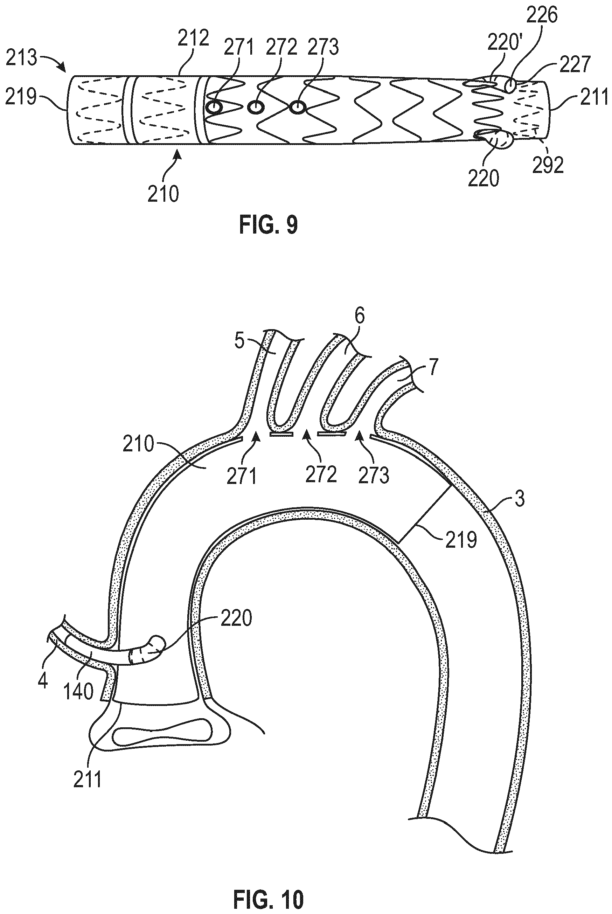

Each of Figure 9 and Figure 10 illustrates another example endoluminal prosthesis 210. Endoluminal prosthesis 210 is similar to endoluminal prosthesis 10 described above and illustrated in Figures 1, 2A, 2B, 2C, 2D, 2E, 2F, 4A, 4B, 4C, 5, 6, 7, and 8, except as detailed below. Thus, endoluminal prosthesis 210 includes a main tubular body 212 defining a main lumen 213 extending between a proximal end 211 and a distal end 219, a first curved side tube 220 extending through main tubular body 212. The side tube 220 defines a side tube lumen 223 having an inlet (not visible in the Figures) and an outlet 226. The inlet is in fluid communication with the main lumen 213. The side tube lumen 223 comprises an internal portion that extends into the main lumen 213 and an external portion 225 external to the main tubular body 212. The external portion of the side tube 220 curves partially around the main tubular body 212, which positions the outlet 226 facing in an approximately circumferential or tangential direction with respect to the main tubular body 212. Endoluminal prosthesis includes a second curved side tube 220' with a similar structure to the first curved side tube 220.

In this embodiment, only one stent 292 is positioned axially between the proximal edge 227 of outlet 226 of side tube 220 and the proximal end 211 of endoluminal prosthesis 210. Furthermore, stent 292 is an internal stent. This structural arrangement is considered advantageous for endoluminal prostheses intended to be deployed in a landing zone of a body vessel that provides minimal space between the intended landing zone for the proximal end 211 of the endoluminal prosthesis 210 and a branch vessel that branches from the body vessel and that is intended to be cannulated through the side tube 220. Also in this embodiment, main tubular body 212 defines a plurality of fenestrations 271, 272, 273, which can be used for cannulation of other branch vessels, such as the brachiocephalic artery 5, the left common carotid artery 6, and the left subclavian artery 7. Fenestrations 271, 272, 273 are exemplary only; any structure suitable for cannulation of branch vessels can be used, including side tubes or other suitable structures.

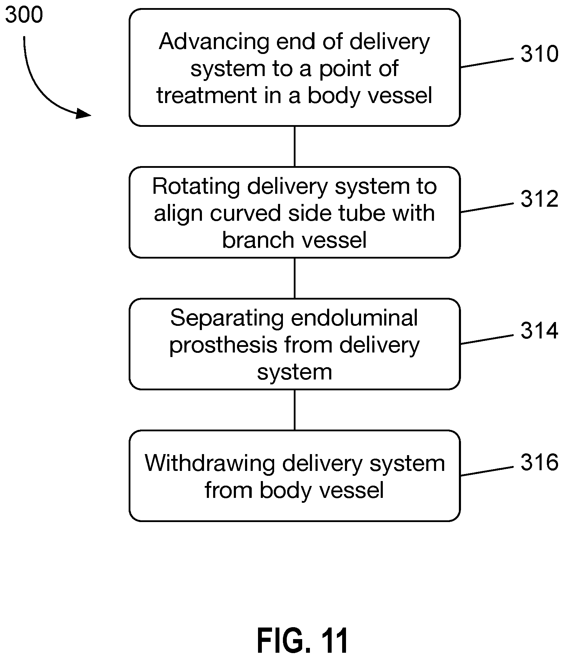

Fig. 11 is a flowchart illustration of an example method 300 of deploying an endoluminal prosthesis at a point of treatment in a body vessel of an animal, such as a human being. Performance of the method 300 results in deployment of a main endoluminal prosthesis at a point of treatment in a body vessel of an animal. An initial step 310 comprises advancing an end of a delivery system on which an endoluminal prosthesis according to an embodiment is disposed to a point of treatment in a body vessel. Another step 312 comprises rotating the delivery system along its longitudinal axis to align one or more curved side tubes of the endoluminal prosthesis with one or more branch vessels of the body vessel. Another step 314 comprises separating the endoluminal prosthesis from the delivery system. Another step 316 comprises withdrawing the delivery system from the body vessel.

In performance of method 300, an endoluminal prosthesis according to any embodiment can be used, including example endoluminal prosthesis 10 and example endoluminal prosthesis 210.

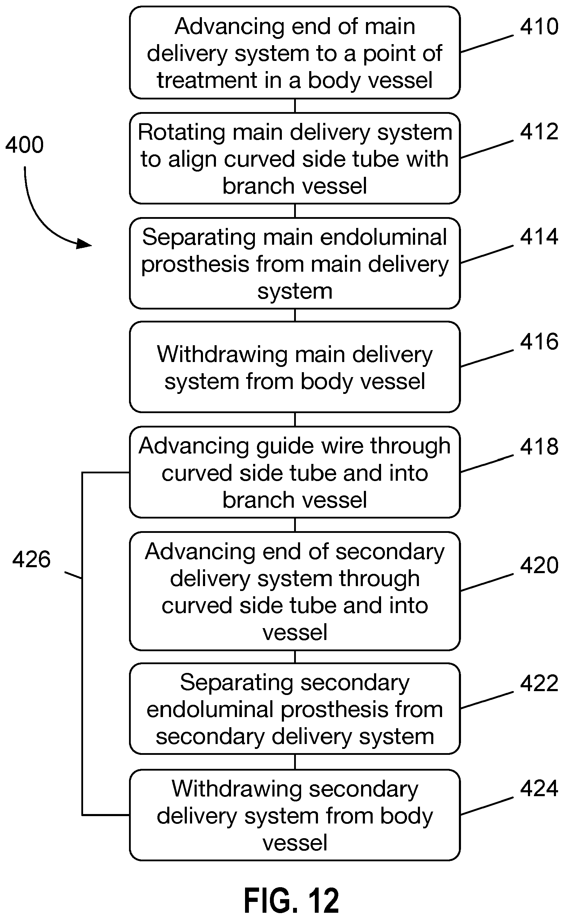

Fig. 12 is a flowchart illustration of an example method 400 of deploying a main endoluminal prosthesis and a secondary endoluminal prosthesis in a body vessel of an animal, such as a human being. Performance of the method 400 results in deployment of a main endoluminal prosthesis at a first point of treatment in a body vessel of an animal and deployment of a secondary endoluminal prosthesis at a second point of treatment in a branch vessel that branches from the body vessel. An initial step 410 comprises advancing an end of a main delivery system on which a main endoluminal prosthesis according to an embodiment is disposed to a point of treatment in the body vessel. Another step 412 comprises rotating the main delivery system along its longitudinal axis to align one or more curved side tubes of the main endoluminal prosthesis with one or more branch vessels of the body vessel. Another step 414 comprises separating the main endoluminal prosthesis from the main delivery system. Another step 416 comprises withdrawing the main delivery system from the body vessel. Another step 418 comprises advancing a guide wire into the lumen of the main endoluminal prosthesis, through the lumen of the curved side tube of the main endoluminal prosthesis, and into the branch vessel. Another step 420 comprises advancing an end of a secondary delivery system on which the secondary endoluminal prosthesis is disposed over the guide wire, through the lumen of the curved side tube of the main endoluminal prosthesis, and into the branch vessel. Another step 422 comprises separating the secondary endoluminal prosthesis from the secondary delivery system. Another step 424 comprises withdrawing the secondary delivery system from the body vessel.

In performance of method 400, an endoluminal prosthesis according to any embodiment can be used, including example endoluminal prosthesis 10 and example endoluminal prosthesis 210. Also, any suitable secondary endoluminal prosthesis can be used, including expandable coronary stent 140. Steps 418, 420, 422, and 424 can be repeated any suitable number of times, using an additional secondary delivery system with an additional secondary endoluminal prosthesis with each repetition, illustrated by optional step 426. If repetition step 426 is included, another, different curved side tube of the main endoluminal prosthesis can be used during performance of each of steps 418 and 420.

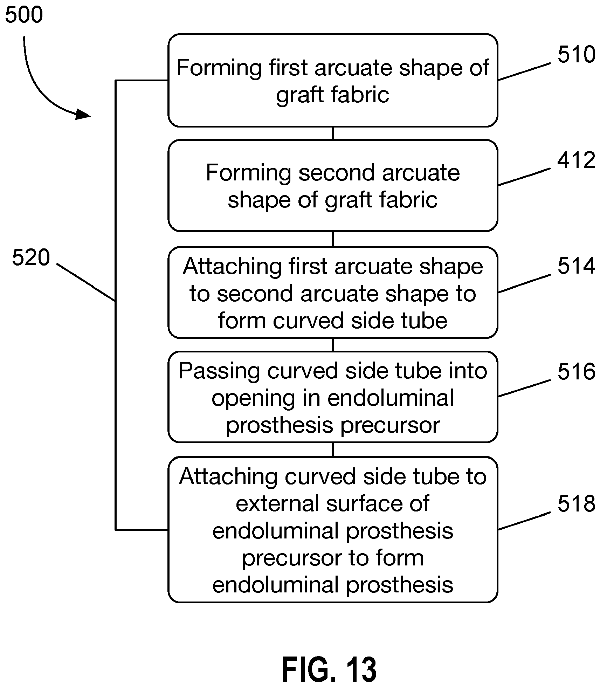

Fig. 13 is a flowchart illustration of an example method 500 of making an endoluminal prosthesis. Performance of method 500 results in an endoluminal prosthesis according to an embodiment, such as example endoluminal prosthesis 10 and example endoluminal prosthesis 210. An initial step 510 comprises forming a first arcuate shape of graft fabric to have a first inner radius and a first outer radius. Another step 512 comprises forming a second arcuate shape of graft fabric to have a second inner radius and a second outer radius. Another step 514 comprises attaching the first arcuate shape to the second arcuate shape along the first and second inner radii and the first and second outer radii to form a curved side tube. Another step 516 comprises passing the curved side tube into an opening in an endoluminal prosthesis precursor such that a portion of the curved side tube is disposed within the lumen of the endoluminal prosthesis precursor and a portion of the curved side tube is external to the lumen of the endoluminal prosthesis precursor. The endoluminal prosthesis precursor can be an endoluminal prosthesis, such as a stent graft, that defines one or more fenestrations, such as an oval- or egg-shaped fenestrations, configured to receive a side tube produced by step 514. Another step 518 comprises attaching the curved side tube to the external surface of the endoluminal prosthesis precursor to form an endoluminal prosthesis having a curved side tube. Advantageously, step 518 is performed such that a first part of the portion of the curved side tube that is external to the lumen of the endoluminal prosthesis is positioned radially inward of, or beneath, a first external stent of the endoluminal prosthesis and a second part of the portion of the curved side tube that is external to the lumen of the endoluminal prosthesis is positioned radially outward of a second external stent of the endoluminal prosthesis. Additional optional steps for inclusion of the method 500 are described above in relation to fabrication of the side tube 20 of example endoluminal prosthesis 10.Steps 510, 512, 514, 516, and 518 can be repeated any suitable number of times, illustrated by optional step 520, forming and attaching an additional curved side tube with each repetition until a desired number of side tubes is attached to the endoluminal prosthesis precursor to form the final endoluminal prosthesis.

Embodiments of the disclosure provide positional flexibility of a branch, whereby the branch does not have to align perfectly with the target vessel. It can also assist in providing a smoother projection toward vessels that are not steeply upward or downward facing.

Embodiments of the disclosure allow a branch to be adapted to suit various anatomies to mimic the same trajectory of the target vessel and offer the smoothest transition.

While the disclosure has been primarily described with reference to having a side tube aligned with a coronary artery, with other embodiments of the disclosure, not shown, similar stent-grafts 10 can be used to stent other branch arteries.

The reference to any prior art in this specification is not, and should not be taken as, an acknowledgement or any form of suggestion that such prior art forms part of the common general knowledge.

In some cases, a single embodiment may, for succinctness and/or to assist in understanding the scope of the disclosure, combine multiple features. It is to be understood that in such a case, these multiple features may be provided separately (in separate embodiments), or in any other suitable combination. Alternatively, where separate features are described in separate embodiments, these separate features may be combined into a single embodiment unless otherwise stated or implied. This also applies to the claims which can be recombined in any combination. That is a claim may be amended to include a feature defined in any other claim. Further a phrase referring to "at least one of" a list of items refers to any combination of those items, including single members. As an example, "at least one of: a, b, or c" is intended to cover: a, b, c, a-b, a-c, b-c, and a-b-c.

Those with ordinary skill in the art will appreciate that various modifications and alternatives for the described and illustrated examples can be developed in light of the overall teachings of the disclosure, and that the various elements and features of one example described and illustrated herein can be combined with various elements and features of another example without departing from the scope of the invention. Accordingly, the particular arrangements of elements and steps disclosed herein have been selected by the inventors simply to describe and illustrate examples of the invention and are not intended to limit the scope of the invention or its protection, which is to be given the full breadth of the appended claims and any and all equivalents thereof.

All optional and preferred features and modifications of the described embodiments and dependent claims and clauses below are usable in all aspects of the invention taught herein. Furthermore, the individual features of the dependent claims and clauses below, as well as all optional and preferred features and modifications of the described embodiments are combinable and interchangeable with one another.

The disclosures in Australian patent application number 2023902394, from which this application claims priority, and in the abstract accompanying this application are incorporated herein by reference.

The skilled person will appreciate that inventive aspects are disclosed herein, including an endoluminal prosthesis as in any of the following clauses.
1. An endoluminal prosthesis, comprising:
   a main tubular body having an internal surface and an external surface and defining a main lumen; and
   at least one curved side tube comprising an internal portion and an external portion and defining a side tube lumen, the internal portion disposed within the main lumen and defining an inlet of the side tube lumen in fluid communication with the main lumen, the external portion extending along the external surface of the main tubular body and defining an outlet of the side tube lumen.
2. The endoluminal prosthesis of clause 1, further comprising a first reinforcement member attached to the inlet of the at least one curved side tube.
3. The endoluminal prosthesis of clause 1 or 2, further comprising a second reinforcement member attached to the outlet of the at least one curved side tube.
4. The endoluminal prosthesis of any preceding clause, wherein an entrance axis extends through the inlet and is positioned at an obtuse angle with respect to a longitudinal axis of the main lumen.
5. The endoluminal prosthesis of any preceding clause, further comprising a first radiopaque marker on the internal portion adjacent to the inlet.
6. The endoluminal prosthesis of any preceding clause, further comprising a second radiopaque marker on the external portion adjacent to the outlet.
7. The endoluminal prosthesis of any preceding clause, further comprising a first intermediate reinforcement member attached to the at least one side tube between the inlet and the outlet.
8. The endoluminal prosthesis as of any preceding clause, further comprising a second intermediate reinforcement member attached to the at least one side tube between the inlet and the outlet.
9. The endoluminal prosthesis of any preceding clause, wherein the at least one curved side tube comprises a first piece of curved graft material having a first inner radius and a first outer radius and a second piece of curved graft material having a second inner radius and a second outer radius;
   wherein the first piece of curved graft material is attached to the second piece of curved graft material along the first and second inner radii and the first and second outer radii.
10. The endoluminal prosthesis of any preceding clause, wherein the external portion extends along a curvilinear path on the external surface of the main tubular body.
11. The endoluminal prosthesis of any preceding clause, wherein the external portion includes a first portion and a second portion extending along the external surface of the main tubular body, the first portion extending in a direction substantially parallel to a longitudinal axis of the main tubular body and the second portion extending from the first portion and along a curvilinear path.
12. The endoluminal prosthesis of any preceding clause, wherein the inlet includes a wire ring having a first circumferential portion attached to the internal surface of the main tubular body.
13. The endoluminal prosthesis of clause 12, wherein the wire ring has a second circumferential portion that is free of the internal surface of the main tubular body.
14. The endoluminal prosthesis of clause 12 or 13, wherein the wire ring is disposed at a non-orthogonal and non-parallel angle to the longitudinal axis of the main tubular body.
15. The endoluminal prosthesis of clause 12, 13, or 14, wherein the wire ring extends proximally from the first circumferential portion to the second circumferential portion.
16. The endoluminal prosthesis of any preceding clause, wherein each of the first inner radius and the second inner radius is between 9 mm and 19 mm.
17. The endoluminal prosthesis of any preceding clause, wherein each of the first outer radius and the second outer radius is between 20 mm and 30 mm.
18. The endoluminal prosthesis of any preceding clause, wherein each of the first inner radius and the second inner radius is between 9 mm and 19 mm; and
   wherein each of the first outer radius and the second outer radius is between 20 mm and 30 mm.
19. An endoluminal prosthesis, comprising:
   a main tubular body having an external surface and defining a main lumen;
   at least one curved side tube comprising an internal portion and an external portion and defining a side tube lumen, the internal portion disposed within the main lumen and defining an inlet of the side tube lumen in fluid communication with the main lumen, the external portion extending along a curvilinear path on the external surface of the main tubular body and defining an outlet of the side tube lumen;
   a first self-expanding stent attached to the external surface of the main tubular body and including a portion disposed radially outward of the external portion of the at least one curved side tube; and
   a second self-expanding stent attached to the main tubular body and including a portion disposed radially inward of the external portion of the at least one curved side tube.
20. An endoluminal prosthesis, comprising:
   a main tubular body having an external surface and defining a main lumen;
   a first curved side tube on a first side of the main tubular body, the first curved side tube comprising a first internal portion and a first external portion and defining a first side tube lumen, the first internal portion disposed within the main lumen and defining a first inlet of the first side tube lumen in fluid communication with the main lumen, the first external portion extending along a first curvilinear path on the external surface of the main tubular body and defining a first outlet of the first side tube lumen;
   a second curved side tube on a second side of the main tubular body, the second curved side tube comprising a second internal portion and a second external portion and defining a second side tube lumen, the second internal portion disposed within the main lumen and defining a second inlet of the second side tube lumen in fluid communication with the main lumen, the second external portion extending along a second curvilinear path on the external surface of the main tubular body and defining a second outlet of the second side tube lumen;
   a first self-expanding stent attached to the external surface of the main tubular body and including a first stent portion disposed radially outward of and attached to the first external portion of the first curved side tube and a second stent portion disposed radially outward of and attached to the second external portion of the second curved side tube; and
   a second self-expanding stent attached to the main tubular body and including a third stent portion disposed radially inward of the first external portion of the first curved side tube and a fourth stent portion disposed radially inward of the second external portion of the second curved side tube.

## Claims

1. An endoluminal prosthesis, comprising:
a main tubular body having an internal surface and an external surface and defining a main lumen; and
at least one curved side tube comprising an internal portion and an external portion and defining a side tube lumen, the internal portion disposed within the main lumen and defining an inlet of the side tube lumen in fluid communication with the main lumen, the external portion extending along the external surface of the main tubular body and defining an outlet of the side tube lumen.

2. The endoluminal prosthesis of claim 1, further comprising a first reinforcement member attached to the inlet of the at least one curved side tube.

3. The endoluminal prosthesis of claim 1 or 2, further comprising a second reinforcement member attached to the outlet of the at least one curved side tube.

4. The endoluminal prosthesis of any preceding claim, wherein an entrance axis extends through the inlet and is positioned at an obtuse angle with respect to a longitudinal axis of the main lumen.

5. The endoluminal prosthesis of any preceding claim, further comprising a first radiopaque marker on the internal portion adjacent to the inlet.

6. The endoluminal prosthesis of any preceding claim, further comprising a second radiopaque marker on the external portion adjacent to the outlet.

7. The endoluminal prosthesis of any preceding claim, further comprising a first intermediate reinforcement member attached to the at least one side tube between the inlet and the outlet.

8. The endoluminal prosthesis as of any preceding claim, further comprising a second intermediate reinforcement member attached to the at least one side tube between the inlet and the outlet.

9. The endoluminal prosthesis of any preceding claim, wherein the at least one curved side tube comprises a first piece of curved graft material having a first inner radius and a first outer radius and a second piece of curved graft material having a second inner radius and a second outer radius;
wherein the first piece of curved graft material is attached to the second piece of curved graft material along the first and second inner radii and the first and second outer radii.

10. The endoluminal prosthesis of any preceding claim, wherein the external portion extends along a curvilinear path on the external surface of the main tubular body.

11. The endoluminal prosthesis of any preceding claim, wherein the external portion includes a first portion and a second portion extending along the external surface of the main tubular body, the first portion extending in a direction substantially parallel to a longitudinal axis of the main tubular body and the second portion extending from the first portion and along a curvilinear path.

12. The endoluminal prosthesis of any preceding claim, wherein the inlet includes a wire ring having a first circumferential portion attached to the internal surface of the main tubular body;
wherein optionally the wire ring has a second circumferential portion that is free of the internal surface of the main tubular body;
wherein optionally the wire ring is disposed at a non-orthogonal and non-parallel angle to the longitudinal axis of the main tubular body; and/or
wherein optionally the wire ring extends proximally from the first circumferential portion to the second circumferential portion.

13. The endoluminal prosthesis of any preceding claim, wherein each of the first inner radius and the second inner radius is between 9 mm and 19 mm; and/or
wherein each of the first outer radius and the second outer radius is between 20 mm and 30 mm.

14. An endoluminal prosthesis according to any preceding claim, wherein the external portion extends along a curvilinear path on the external surface of the main tubular body; the endoluminal prosthesis comprising:
a first self-expanding stent attached to the external surface of the main tubular body and including a portion disposed radially outward of the external portion of the at least one curved side tube; and
a second self-expanding stent attached to the main tubular body and including a portion disposed radially inward of the external portion of the at least one curved side tube.

15. An endoluminal prosthesis according to any preceding claim, comprising:
a first curved side tube on a first side of the main tubular body, the first curved side tube comprising a first internal portion and a first external portion and defining a first side tube lumen, the first internal portion disposed within the main lumen and defining a first inlet of the first side tube lumen in fluid communication with the main lumen, the first external portion extending along a first curvilinear path on the external surface of the main tubular body and defining a first outlet of the first side tube lumen;
a second curved side tube on a second side of the main tubular body, the second curved side tube comprising a second internal portion and a second external portion and defining a second side tube lumen, the second internal portion disposed within the main lumen and defining a second inlet of the second side tube lumen in fluid communication with the main lumen, the second external portion extending along a second curvilinear path on the external surface of the main tubular body and defining a second outlet of the second side tube lumen;
a first self-expanding stent attached to the external surface of the main tubular body and including a first stent portion disposed radially outward of and attached to the first external portion of the first curved side tube and a second stent portion disposed radially outward of and attached to the second external portion of the second curved side tube; and
a second self-expanding stent attached to the main tubular body and including a third stent portion disposed radially inward of the first external portion of the first curved side tube and a fourth stent portion disposed radially inward of the second external portion of the second curved side tube.
